**Europäisches Patentamt**

**European Patent Office**

**Office européen des brevets**

(19)

(11) Veröffentlichungsnummer: **0 168 786**
**A2**

(12)
# EUROPÄISCHE PATENTANMELDUNG

(21) Anmeldenummer: **85108713.0**

(22) Anmeldetag: **12.07.85**

(51) Int. Cl.⁴: **G 01 N 33/00**

(30) Priorität: **17.07.84 DE 3426329**

(43) Veröffentlichungstag der Anmeldung: **22.01.86**
**Patentblatt 86/4**

(84) Benannte Vertragsstaaten: **AT CH DE FR GB IT LI**

(71) Anmelder: **Bernath, Tibor, Distelborn 6,
D-3000 Hannover 91 (DE)**

(72) Erfinder: **Bernath, Tibor, Distelborn 6,
D-3000 Hannover 91 (DE)**

(74) Vertreter: **Wuesthoff, Franz, Dr.-Ing. et al, Patentanwälte
Wuesthoff -v. Pechmann-Behrens-Goetz
Schweigerstrasse 2, D-8000 München 90 (DE)**

(54) **Vorrichtung zum Messen von Gaskonzentrationen in einer aus einer Prozesskammer entnommenen heissen Gasprobe.**

(57)   Vorrichtung zum Messen einer Gaskonzentration in einer aus einer Prozeßkammer entnommenen heißen Gasprobe, bei der ein temperierbarer Metallblock (10) vorgesehen ist, welcher zumindest den Detektor (12) und die meßgasführenden Teile im Strömungsweg zwischen einer Entnahmesonde und dem Detektor aufnimmt. Eine Saugpumpe (14) saugt die Gasprobe in den Metallblock (10), wo das Meßgas in zwei Leitungen aufgeteilt wird. Die erste Leitung führt zum Detektor und die zweite Leitung zurück zur Prozeßkammer.

0168786

PATENTANWÄLTE

# WUESTHOFF - v. PECHMANN - BEHRENS - GOETZ

EUROPEAN PATENT ATTORNEYS

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING.; DIPL.-WIRTSCH.-ING. RUPERT GOETZ

D-8000 MÜNCHEN 90
SCHWEIGERSTRASSE 2

TELEFON: (089) 66 20 51
TELEGRAMM: PROTECTPATENT
TELEX: 5 24 070
TELEFAX: VIA (089) 271 60 63 (III)

Tibor Bernath
EP-59 481

### Vorrichtung zum Messen von Gaskonzentrationen in einer aus einer Prozesskammer entnommenen heißen Gasprobe

Die Erfindung betrifft eine Vorrichtung zum Messen insbesondere der Kohlenwasserstoffkonzentration einer aus einer Prozesskammer entnommenen heißen Gasprobe und zum Erzeugen eines der Konzentration entsprechenden elektrischen Überwachungssignales mit einer in die Prozesskammer einführbaren, temperierten Entnahmesonde, einem außerhalb der Prozesskammer, benachbart der Prozesskammerwand angeordneten Kohlenwasserstoff-Detektor , wie z.B. ein Flammenionisationsdetektor, einer Saugpumpe für die Entnahme der Gasprobe und mit einem die genannten Teile außerhalb der Prozesskammer umschließenden, thermisch isolierenden Gehäuse. Derartige Vorrichtungen finden beispielsweise Verwendung zur Überwachung der Kohlenwasserstoffkonzentration in Trocknerofenkammern von Industrielackierereien. Die Konzentration an Kohlenwasserstoffen darf in solchen Prozesskammern keine bestimmten Werte überschreiten, wenn die Gefahr einer Explosion vermieden sein soll. Wird mittels der Meßvorrichtung ein Anstieg der Konzentration über den zulässigen Wert festgestellt, so wird die Anlage außer Betrieb gesetzt.

Die aus der Prozesskammer entnommene Gasprobe hat häufig eine Temperatur von 250° C oder mehr, so daß sichergestellt sein muß, daß auf dem Transportweg zum Detektor das Gas nicht mit kälteren Oberflächen in Berührung kommt, an denen die organischen Dämpfe kondensieren können. Aus diesem Grunde wird der Kohlenwasserstoff-Detektor bevorzugt benachbart der Prozesskammerwand angeordnet, so daß sich ein möglichst kurzer Transportweg für die Gasprobe von der Kammer zum Detektor ergibt. Um eine Kondensation der organischen Substanzen zu vermeiden, welche das Meßergebnis erheblich verfälschen würde, müssen sämtliche mit der Gasprobe in Berührung kommende Teile gleichmäßig und ohne zeitliche Verzögerungen temperiert werden, was Schwierigkeiten bereitet. Insbesondere dann, wenn die Gasprobe nach Passieren der Entnahmesonde in eine Analysenkammer geführt wird, wo die gasführenden Teile mit heißer Luft temperiert werden, ist keine homogene Temperaturverteilung der einzelnen Bauteile gewährleistet, da Luft eine schlechte Wärmeleitfähigkeit aufweist.

Ein weiterer Nachteil bekannter Vorrichtungen dieser Art liegt darin, daß sich nach der Analyse der Gasprobe am Detektor beim Gasaustritt Kondensat bildet.

Der Erfindung liegt die Aufgabe zugrunde, die Vorrichtung der eingangs genannten Art derart weiterzubilden, daß die mittels des Detektors gemessene Kohlenwasserstoffkonzentration der Gasprobe derjenigen in der Prozesskammer genau entspricht.

Gemäß der Erfindung wird diese Aufgabe dadurch gelöst, daß in dem Gehäuse ein temperierbarer, massiver Metallblock vorgesehen ist, welcher zumindest den Detektor und die meßgasführenden Teile im Strömungsweg zwischen der Entnahmesonde und dem Detektor aufnimmt, wobei die Strömungswege als Bohrungen im Metallblock ausgebildet sind.

- 3 -

In einer bevorzugten Ausgestaltung der Erfindung ist vorgesehen, daß der Metallblock auch die Saugpumpe aufweist, so daß diese im Transportweg der Gasprobe zwischen Kammer und Detektor angeordnet werden kann.

Dadurch, daß erfindungsgemäß der temperierbare Metallblock, welcher aufgrund der guten Wärmeleitfähigkeit des Metalles eine homogene Temperaturverteilung aufweist, alle meßgasführenden Teile im Strömungsweg zwischen der Entnahmesonde und dem Detektor aufnimmt, ist gewährleistet, daß alle Teile, mit denen das Meßgas in Berührung kommt, die Temperatur des Metallblockes haben, so daß keine das Meßergebnis verfälschenden Kondensationen auftreten können.

Der Metallblock läßt sich integral, einstückig ausformen, wobei die einzelnen Bauteile, wie der Detektor, die Saugpumpe, ein Gasfilter etc. in Bohrungen im Metallblock einschiebbar sind.

In einer bevorzugten Ausgestaltung der Erfindung ist vorgesehen, daß der Metallblock aus zwei trennbaren Elementen besteht, von denen das eine den Detektor und das andere die Saugpumpe aufnimmt. Die beiden Elemente sind mit gutem thermischen Kontakt miteinander verbindbar, so daß ein einziger Heizkörper ausreicht, um den gesamten Metallblock gleichmäßig zu erhitzen.

Die zur Erhitzung erforderliche Heizleistung läßt sich dadurch reduzieren, daß der Metallblock mit einer Rundum-Isolierung versehen ist.

– 4 –

Als Detektor dient bei einer erfindungsgemäßen Vorrichtung üblicherweise ein bekannter Flammenionisationsdetektor, bei dem an eine reine Wasserstoffflamme, die unter Zufuhr von kohlenwasserstofffreier Luft brennt, ein elektrisches Feld angelegt wird. Die Gasprobe wird in die Flamme eingeführt, wobei es zu einer erheblichen Ionenbildung kommt, welche im angelegten elektrischen Feld einen Stromfluß verursacht. Das verstärkte Stromsignal ist ein Maß für die Konzentration der Kohlenwasserstoffe in der Gasprobe.

Die in die Flamme zu leitende Gasmenge ist relativ gering und beträgt typischerweise etwa 20 ml/min.

In einer bevorzugten Ausgestaltung der Erfindung wird zur Erreichung eines genauen Meßergebnisses eine wesentlich größere Gasmenge mittels der Saugpumpe aus der Prozesskammer entnommen und auf der Druckseite der Saugpumpe das Meßgas in zwei Leitungen geteilt, wobei eine erste Leitung zum Detektor führt und eine zweite Leitung zurück zur Prozesskammer. Die Gasströmung in der zweiten Leitung ist wesentlich größer als in der ersten Leitung, welche das Gas zur Flamme führt.

Um stabile Strömungsverhältnisse zu erreichen, sind die leitungen jeweils mit Kapillaren versehen.

In einer bevorzugten Weiterbildung der Erfindung wird die Sicherheit der Meßvorrichtung dadurch erhöht, daß auf der Druckseite der Saugpumpe ein Druckschalter vorgesehen ist, welcher bei Abfallen des Pumpen-Staudruckes ein Störungssignal abgibt.

- 5 -

Zur Stabilisierung des Druckes auf der Druckseite der Saugpumpe und zur Konstanthaltung der Gasströmung in der ersten Leitung zur Detektor-Flamme ist in einer Ausgestaltung der Erfindung ein Steuerluftsystem vorgesehen, welches mittels eines Druckminderers für eine konstante Strömung in der zweiten Leitung sorgt.

Zur zuverlässigen und wiederholbaren Kalibrierung des Detektors ist in einer bevorzugten Weiterbildung der Erfindung vorgesehen, daß neben den Anschlüssen für das Brenngas ($H_2$) der Flamme und die Brennluft auch jeweils ein Anschluß für ein kohlenwasserstofffreies Gas und für ein Gas mit einer definierten Kohlenwasserstoffkonzentration mit der Flamme leitend verbindbar sind. Bei dem kohlenwasserstofffreien Gas kann es sich um saubere Luft handeln.

Eine lückenlose und vollautomatische Eigenüberwachung der Meßvorrichtung wird in einer Weiterbildung der Erfindung dadurch erreicht, daß dann, wenn die entnommene Gasprobenmenge einen vorbestimmten Wert unterschreitet, ein Signal ausgelöst wird. Dadurch ist gewährleistet, daß in der Flamme des Detektors ständig Gas aus der Prozesskammer analysiert wird.

Die Sicherheit der Meßvorrichtung läßt sich auch dadurch erhöhen, daß das Ausgangssignal des Detektors auf einen Sollwert überwacht wird, um bei einem Ausfall z.B. des Detektors oder der Saugpumpe ein Alarmsignal abzugeben.

Nachfolgend ist die Erfindung anhand eines Ausführungsbeispieles näher beschrieben. Dabei zeigt:

Fig. 1 eine Explosionsdarstellung einer erfindungsgemäßen Meßvorrichtung;

Fig. 2  eine Explosionsdarstellung des Metallblockes der
        Meßvorrichtung im Detail und

Fig. 3  schematisch das Gas-Leitungssystem der Meßvorrichtung.

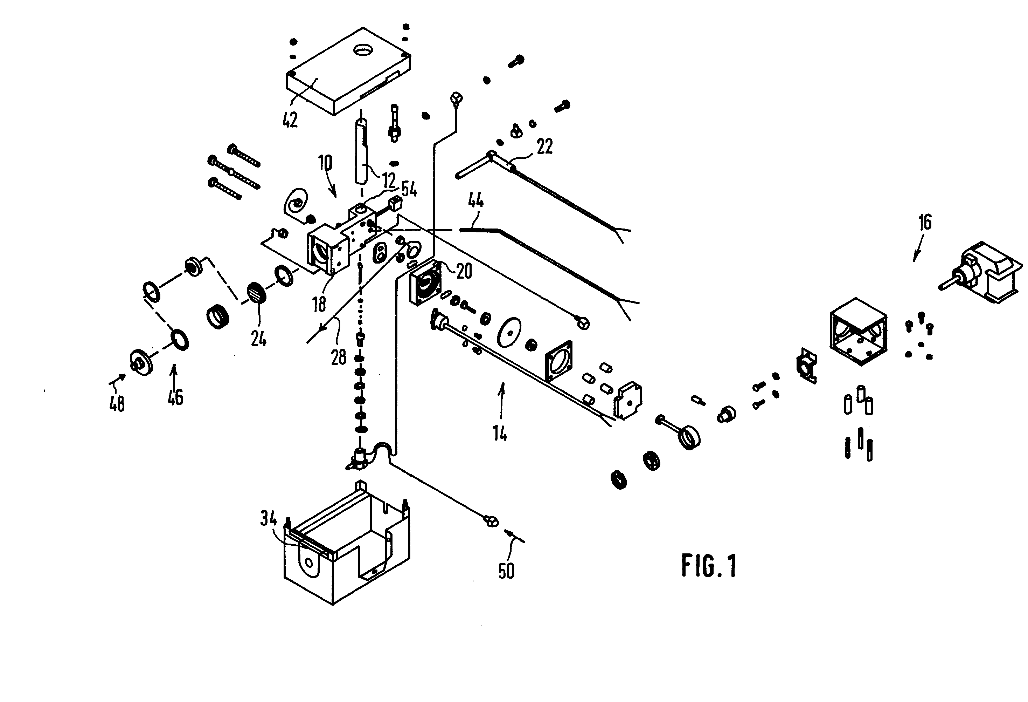

Fig. 1 zeigt den Metallblock 10 der Meßvorrichtung, in
welchen der Detektor 12 (ein Flammenionisationsdetektor)
eingeschoben wird. Der Metallblock 10 wird direkt benachbart der Wand der Prozesskammer (nicht gezeigt) angebracht
und ist mit einer Saugpumpe 14 versehen, welche über eine
Entnahmesonde (nicht gezeigt) die Gasprobe aus der Prozesskammer in den Metallblock 10 saugt. Der Antrieb der Saugpumpe 14 ist in Fig. 1 mit dem Bezugszeichen 16 versehen.

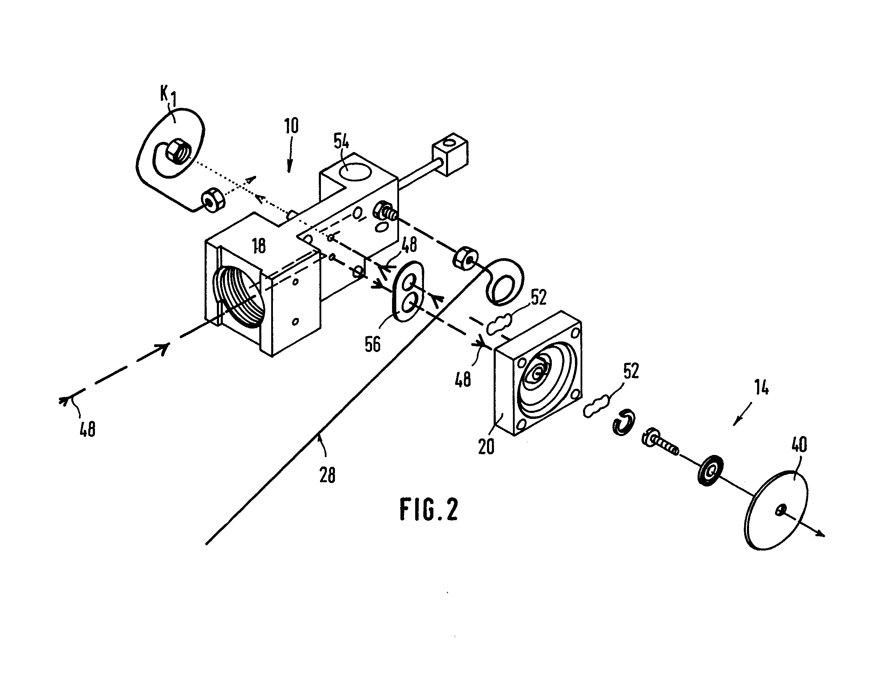

Der Metallblock 10 ist gemäß den Fig. 1 und 2 in zwei
Elemente 18 und 20 aufgeteilt, welche mittels Schrauben
in gutem Wärmekontakt miteinander verbindbar sind. Ein
Heizkörper 22 ist in das Metallblock-Element 18 einschiebbar
und heizt den gesamten Metallblock homogen auf.

Ein Gasfilter 24 ist mittels Dichtungen und Klemmringen 46
im Metallblock 10 befestigt und liegt eingangs des
Strömungsweges des Meßgases, welcher durch den Pfeil 48
angedeutet ist. Das Meßgas tritt also gemäß Fig. 1 in
Richtung des Pfeiles 48 in den Metallblock 10 ein.

Fig. 2 zeigt den weiteren Verlauf des Strömungsweges des
Meßgases durch die Vorrichtung, welcher durch den Pfeil 48
angedeutet ist. Das Meßgas wird durch die im Metallblock-
Element 20 untergebrachte Saugpumpe 14, die als Membranpumpe mit der Membran 40 ausgebildet ist, durch den Metallblock über die Kapillare $K_1$ zum Flammendetektor 12 geführt,
welcher in die Bohrung 54 im Metallblock 10 eingeschoben
ist. In Fig. 1 ist eine Rundum-Isolierung 34 mit einer oberen Abdeckung 42 für den Metallblock 10 gezeigt, welche die vom Heizkörper 22 zu erbringende Heizleistung reduziert. Mittels eines Temperaturfühlers 44 wird die Temperatur des Metallblockes 10 ermittelt. Die beiden Elemente 18, 20 des Metallblockes 10 sind mittels einer Dichtung 56 derart miteinander verbindbar, daß das Metallblock-Element 20 ohne Verzögerung die Temperatur des Metallblock-Elementes 18 annimmt.

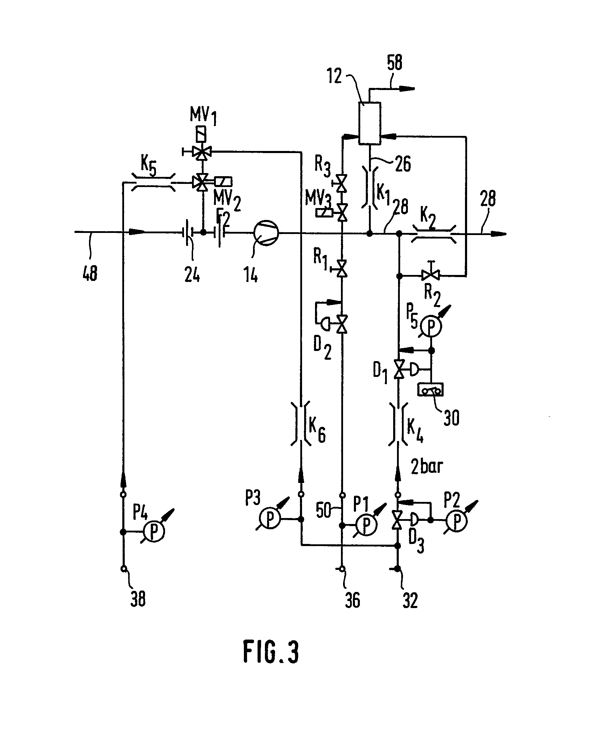

In Fig. 3 sind die Strömungsverhältnisse des Meßgases sowie der übrigen Gase schematisch dargestellt. Das Meßgas tritt gemäß dem Pfeil 48 (siehe auch Fig. 1 und 2) über das Filter 24 unter der Wirkung der Saugpumpe 14 in den Metallblock 10 ein. Auf der Druckseite der Saugpumpe 14 wird der Gasstrom in 2 Anteile aufgeteilt. In die Leitung 26, welche die Kapillare $K_1$ beinhaltet, fließt ein geringer Gasstrom von etwa 20 ml/min zum Detektor 12, wo er der Flamme des Flammenionisationsdetektors zugeführt wird. Der größere Anteil des angesaugten Gasstromes von etwa 1,6 l/min fließt über die Leitung 28 mit der Kapillare $K_2$ zurück zur Prozesskammer (nicht gezeigt).

Die zum Detektor 12 gelangende Gasmenge hängt vom Differenzdruck an der Kapillare $K_1$ ab. Die Sekundärseite der Kapillare $K_1$ ist mit dem Detektor 12 verbunden und steht praktisch unter atmosphärischem Druck. Demzufolge ist die durch die Kapillare $K_1$ strömende Gasmenge vorwiegend eine Funktion des primärseitigen Druckes, welcher vom Staudruck der Saugpumpe 14 bestimmt wird. Dieser Staudruck der Saugpumpe kann sich jedoch mit einigen Meßgrößen, wie dem Meßgas-Filterwiderstand, der Pumpenförderleistung etc. verändern. Zur Stabilisierung der durch die Kapillare $K_1$ strömenden

Gasprobenmenge ist deshalb ein Luftsteuersystem eingebaut. Das System besteht im wesentlichen aus der Kapillare $K_4$ und dem Druckminderer $D_1$. Die Kapillare $K_2$ ist so dimensioniert, daß bei einer Pumpenleistung von ca. 1,6 l/min und bei einer durch den Druckminderer $D_1$ eingestellten Steuerluftmenge von ca. 0,8 l/min ein Staudruck von ca. 0,2 bar entsteht. Fällt die Fördermenge der Saugpumpe 14 auf ein Minimum von ca. 0,8 l/min ab, so regelt der Druckminderer $D_1$ automatisch nach und stellt den Staudruck von 0,2 bar her. Fällt die Fördermenge der Saugpumpe weiter ab, so versucht der Druckminderer $D_1$ weiterhin nachzuregeln. Dazu sind jedoch immer größere Durchflußmengen durch die Kapillare $K_4$ erforderlich. Bei einem bestimmten Druckabfall hinter der Saugpumpe strömt nicht mehr genügend Gas durch die Kapillare $K_4$ , so daß der Mindesteingangsdruck am Druckminderer $D_1$ nicht mehr erreicht wird, so daß der Staudruck hinter der Saugpumpe deutlich absinkt. Diese Druckminderung wird vom Druckschalter 30 erfaßt und als Störung gemeldet.

Mit den Bezugszeichen $K_1$ bis $K_5$ sind in Fig. 3 allgemein Kapillaren bezeichnet, welche in den einzelnen Leitungen angeordnet sind. Die Bezugszeichen $P_1$ bis $P_5$ bezeichnen Druckmeßgeräte, welche den Gasdruck an verschiedenen Stellen des Leitungssystems ermitteln. Mit den Bezugszeichen $R_1$ bis $R_3$ sind einzelne Ventile versehen, während Magnetventile mit den Bezugszeichen $MV_1$ bis $MV_3$ bezeichnet sind.

Die Kalibrierung der Meßvorrichtung, also die Zuordnung des mittels des Flammenionisationsdetektors gemessenen Stromes zu einer bestimmten Kohlenwasserstoffkonzentration im Probengas, wird nachfolgend anhand der Fig. 3 beschrieben. Die Vorrichtung verfügt über 3 Gasanschlüsse 32, 36 und 38 (neben dem Anschluß für die Gasprobe entsprechend dem Pfeil 48). Über den Anschluß 32 kann Preßluft in die Vorrichtung dosiert werden, welche einerseits als Brennluft für den kontinuierlichen Betrieb der Flamme dient und andererseits auch als kohlenwasserstofffreie Luft zur Kalibrierung des Detektors 12 geeignet ist. Über den Anschluß 36 gelangt das Brenngas der Flamme ($H_2$) in die Vorrichtung, während über den Anschluß 38 ein sogenanntes Eichgas, also ein Gas mit einer definierten Kohlenwasserstoffkonzentration, der Flamme des Detektors zuführbar ist. Die Kalibrierung des Detektors erfolgt durch sukzessive Aufschaltung zweier Vergleichsgase, nämlich dem kohlenwasserstofffreien Gas und dem Eichgas. Die Aufschaltung der Gase erfolgt jeweils durch Betätigung der Magnetventile $MV_1$ bzw. $MV_2$. Die Dosierung der Kalibriergase erfolgt derart, daß mehr Gas in die Vorrichtung eingegeben wird, als die Saugpumpe fördern kann. Das überschüssige Gas strömt rückwärts gegen die Richtung des Pfeiles 48 in die Prozesskammer.

Das Ventil $R_2$ dient der Einstellung der Brennluftmenge. Der Druckminderer $D_2$ und die Ventile $R_1$ und $R_3$ dienen der Einstellung und der Konstanthaltung der Brenngas-Strömung zur Flamme. Das Brenngas strömt über den Anschluß 36 und die Leitung 50.

2064

0168786

PATENTANWÄLTE

WUESTHOFF - v. PECHMANN - BEHRENS - GOETZ

EUROPEAN PATENT ATTORNEYS

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING. DIPL.-WIRTSCH.-ING. RUPERT GOETZ

D-8000 MÜNCHEN 90
SCHWEIGERSTRASSE 2

TELEFON: (089) 66 20 51
TELEGRAMM: PROTECTPATENT
TELEX: 5 24 070
TELEFAX: VIA (089) 27 16 0 63 (III)

Tibor Bernath
EP-59 481

P a t e n t a n s p r ü c h e :

1. Vorrichtung zum Messen insbesondere der Kohlenwasserstoffkonzentration in einer aus einer Prozesskammer entnommenen heißen Gasprobe und zum Erzeugen eines der Konzentration
entsprechenden elektrischen Überwachungssignales mit einer
in die Prozesskammer einführbaren, temperierten Entnahmesonde, einem außerhalb der Prozesskammer, benachbart der Prozesskammerwand, angeordneten Kohlenwasserstoff-Detektor (12),
wie z.B. ein Flammenionisationsdetektor, einer Saugpumpe (14)
für die Entnahme der Gasprobe und mit einem die genannten
Teile außerhalb der Prozesskammer umschließenden, thermisch
isolierenden Gehäuse (34),
d a d u r c h   g e k e n n z e i c h n e t ,
daß in dem Gehäuse (34) ein temperierbarer, massiver Metallblock (10) vorgesehen ist, welcher zumindest den Detektor
(12) und die meßgasführenden Teile im Strömungsweg zwischen
der Entnahmesonde und dem Detektor (12) aufnimmt, wobei die
Strömungswege als Bohrungen im Metallblock (10) ausgebildet
sind.

2. Vorrichtung nach Anspruch 1,
d a d u r c h   g e k e n n z e i c h n e t ,
daß der Metallblock (10) auch die Saugpumpe (14) aufnimmt.

/2

3. Vorrichtung nach einem der Ansprüche 1 oder 2,
dadurch gekennzeichnet,
daß der Metallblock (10) integral, einstückig ausgeformt ist.

4. Vorrichtung nach einem der Ansprüche 1 oder 2,
dadurch gekennzeichnet,
daß der Metallblock (10) aus zumindest zwei trennbaren
Elementen (18, 20) besteht, von denen das eine (18) den
Detektor (12) und das andere (20) die Saugpumpe (14)
aufnimmt.

5. Vorrichtung nach Anspruch 4,
dadurch gekennzeichnet,
daß die Metallblock-Elemente (18, 20) mit gutem thermischen
Kontakt miteinander verbindbar sind.

6. Vorrichtung nach einem der vorhergehenden Ansprüche,
dadurch gekennzeichnet,
daß zumindest ein elektrisch betreibbarer Heizkörper (22)
in den Metallblock (10) einschiebbar ist.

7. Vorrichtung nach einem der vorhergehenden Ansprüche,
dadurch gekennzeichnet,
daß ein Gasfilter (24) im Metallblock aufgenommen ist.

8. Vorrichtung nach einem der vorhergehenden Ansprüche,
dadurch gekennzeichnet,
daß als Saugpumpe (14) eine Membranpumpe vorgesehen ist.

9. Vorrichtung nach einem der vorhergehenden Ansprüche,
dadurch gekennzeichnet,
daß auf der Druckseite der Saugpumpe (14) das Meßgas in zwei
Leitungen (26, 28) geteilt wird, wobei eine erste Leitung
(26) zum Detektor (12) führt und eine zweite Leitung (28)
zur Prozesskammer.

10. Vorrichtung nach Anspruch 9,
dadurch gekennzeichnet,
daß die Gasströmung in der zweiten Leitung (28) wesentlich
größer ist als in der ersten Leitung (26).

11. Vorrichtung nach einem der Ansprüche 9 oder 10,
dadurch gekennzeichnet,
daß die erste und/oder die zweite Leitung (26, 28) jeweils eine
Kapillare ($K_1$, $K_2$) aufweisen.

12. Vorrichtung nach einem der vorhergehenden Ansprüche,
dadurch gekennzeichnet,
daß auf der Druckseite der Saugpumpe ein Druckschalter (30)
vorgesehen ist, welcher bei Abfallen des Pumpen-Staudruckes
ein Störungssignal abgibt.

13. Vorrichtung nach Anspruch 12,
dadurch gekennzeichnet,
daß zur Stabilisierung des Druckes auf der Druckseite der
Saugpumpe (14) und zur Konstanthaltung der Gasströmung
in der ersten Leitung (26) ein Steuerluftsystem (32, $K_4$) vorgesehen ist, welches einen Druckminderer ($D_1$) aufweist.

14. Vorrichtung nach einem der vorhergehenden Ansprüche,
dadurch gekennzeichnet,
daß der Metallblock (10) mit einer Rundum-Isolierung (34)
versehen ist.

15. Vorrichtung nach einem der vorhergehenden Ansprüche,
dadurch gekennzeichnet,
daß neben den Anschlüssen für das Brenngas (36) des
Flammenionisationsdetektors, vorzugsweise $H_2$, und die
Brennluft (32) auch ein Anschluß für ein kohlenwasserstofffreies Gas und ein Anschluß (38) für ein Gas mit einer
definierten Kohlenwasserstoffkonzentration vorgesehen sind.

16. Vorrichtung nach einem der vorhergehenden Ansprüche,
dadurch gekennzeichnet,
daß bei Unterschreiten der entnommenen Gasprobenmenge um
einen vorbestimmten Wert ein Signal ausgelöst wird.

17. Vorrichtung nach einem der vorhergehenden Ansprüche,
dadurch gekennzeichnet,
daß das Ausgangssignal des Detektors (12) auf einen Sollwert
überwacht wird, um bei einem Ausfall z.B. des Detektors (12)
oder der Saugpumpe (14) ein Alarmsignal abzugeben.

2064

FIG.1

0168786

0168786

FIG.2

FIG.3